# EUROPEAN PATENT APPLICATION

(11) **EP 4 129 409 A1**
(43) Date of publication of application: **08.02.2023**
(21) Application number: 21776556.9
(22) Date of filing: 25.03.2021
(51) Int. Cl.: A61P 43/00, A61P 17/14, A61Q 7/00, A23L 33/175, A61K 8/44, A61K 8/49, A61K 31/198, A61K 31/405

(54) **COMPOSITION FOR PROMOTING HAIR THICKENING, COMPOSITION FOR SUSTAINING GROWTH PHASE IN HAIR CYCLE AND COMPOSITION FOR PROMOTING VEGF PRODUCTION, COMPOSITION FOR PROMOTING TRANSITION FROM RESTING PHASE TO GROWTH PHASE IN HAIR CYCLE AND COMPOSITION FOR PROMOTING FGF7 PRODUCTION, AND COMPOSITION FOR PROMOTING HAIR CYCLE NORMALIZATION**

(30) Priority: 26.03.2020 JP 2020056850
(71) Applicant: AJINOMOTO CO., INC., Chuo-ku Tokyo 104-8315 (JP)
(72) Inventor: KATAYAMA, Yukino, Kawasaki-shi, Kanagawa 210-8681 (JP); SUZUKI, Kana, Kawasaki-shi, Kanagawa 210-8681 (JP); SUZUKI, Katsuya, Kawasaki-shi, Kanagawa 210-8681 (JP); TANAKA, Chie, Kawasaki-shi, Kanagawa 210-8681 (JP); TAKINO, Yoshinobu, Kawasaki-shi, Kanagawa 210-8681 (JP); SUGA, Yasuyo, Kawasaki-shi, Kanagawa 210-8681 (JP); ICHIKAWA, Reiko, Kawasaki-shi, Kanagawa 210-8681 (JP); AKIYOSHI, Shinobu, Kawasaki-shi, Kanagawa 210-8681 (JP)
(74) Representative: Strehl Schübel-Hopf & Partner
(86) International application number: PCT/JP2021/012524
(87) International publication number: WO 2021/193820

(57) **Abstract**

According to the present invention, a composition that has high safety, can be continuously used for a long period, promotes production of VEGF and FGF7 involved in hair growth, and affords good prophylactic or improving effect on thinning hair or hair loss can be provided.

The present invention relates to a composition for promoting hair thickening, containing one or more members selected from the group consisting of citrulline, tryptophan, glutamine, and ornithine, a composition for maintaining the anagen phase in the hair cycle and a composition for promoting VEGF production, each containing one or more members selected from the group consisting of citrulline, tryptophan, and glutamine, a composition for promoting transition from the telogen phase to the anagen phase in the hair cycle and a composition for promoting FGF7 production, each containing ornithine, and a composition for promoting normalization of the hair cycle, containing one or more members selected from the group consisting of citrulline, tryptophan, glutamine, and ornithine.

## Description

### [Technical Field]

The present invention relates to a composition for promoting hair thickening. In addition, the present invention relates to a composition for maintaining the anagen phase of the hair cycle, a composition for promoting VEGF production, a composition for promoting transition from the telogen phase to the anagen phase of the hair cycle, a composition for promoting FGF7 production, and a composition for promoting normalization of the hair cycle.

### [Background Art]

Many people have long been troubled by thinning hair and hair loss. Hair loss is known to occur due to various causes, and androgenetic alopecia caused by the action of androgen and female pattern alopecia caused by aging and reduction of female hormone are frequently observed alopecia.

Regarding hair growth, the hair cycle consisting of the anagen phase in which hair matrix cells actively differentiate and proliferate after new hair growth until the growth stops, the catagen phase which is the process of transition to the telogen phase after the end of the anagen phase, and the telogen phase during which the hair root degenerates to reach hair loss and thereafter hair growth begins anew is known. Abnormality of this hair cycle is known to be one of the causes of alopecia.

Specifically, it has been reported that female pattern alopecia develops because transition from the telogen phase to the anagen phase of the hair cycle does not occur, and the new hair growth is suppressed, causing thinning hair, and that androgenetic alopecia develops because the anagen phase is shortened in the hair cycle, resulting in thin short hair.

Agents for new hair growth and agents for hair growth, for treating or improving androgenetic alopecia and female pattern alopecia, those containing various active ingredients are currently on the market.

Known active ingredients for external use include carpronium chloride having a blood circulation promoting action, trans-3,4'-dimethyl-3-hydroxyflavanone (t-flavanone) having a proliferation promoting action on hair matrix cells, and adenosine, cytopurine (6-benzylaminopurine), pentadecanoic acid glyceride, and the like having a new hair growth-promoting action. Particularly, the effectiveness of minoxidil which is an antihypertensive agent has been reported.

As oral medicines, it has been reported that finasteride and dutasteride, which are testosterone 5-α reductase inhibitors, are effective for androgenetic alopecia.

However, the improving effects of the above-mentioned components, which are externally applied in anticipation of blood circulation promoting action, hair matrix cell proliferation promoting action, new-hair-growth promoting action, and the like on hair loss and thinning hair are not sufficient. Minoxidil with reported effectiveness has been reported to cause skin symptoms such as pruritus, erythema, desquamation, hair follicle inflammation, contact dermatitis, and the like.

In addition, finasteride and dutasteride cannot be expected to be effective for female pattern alopecia since androgen is not involved.

The effects of amino acids which are components derived from living organisms, to cause hair growth or new hair growth have also been examined, and it has been reported that metabolic intermediates of the urea cycle such as arginine, ornithine, citrulline, argininosuccinic acid, and the like and derivatives thereof can be preferably used in external compositions for increasing, enhancing, or maintaining hair growth (Patent Literature 1).

In addition, a hair treatment composition containing a substance that promotes new hair growth and contains a protein biosynthesis stimulation substance selected from soybean peptide, amino acid, glutamine, glutamic acid, and protein hydrolysate extract, and an antidandruff agent is disclosed (Patent Literature 2).

However, with regard to citrulline and ornithine, which are intermediates of the urea cycle, Patent Literature 1 only shows the observation results of hair growth (hair length increment) in isolated and cultured hair follicles.

In addition, Patent Literature 2 does not specifically indicate the new-hair-growth promoting effect of amino acids themselves, and their effectiveness is unknown.

To promote healthy hair growth and improve thinning hair and hair loss, it is considered effective not only to promote hair length increment and new hair growth, but also to normalize the hair cycle and to thicken hair, that is, to promote the thickening of hair.

### [Citation List]

### [Patent Literature]

[PTL 1]
   Japanese Translation of PCT Application Publication No. h8-502509
[PTL 2]
   Japanese Translation of PCT Application Publication No. 2011-500760

### [Summary of Invention]

### [Technical Problem]

Therefore, the present invention took note of vascular endothelial growth factor (VEGF) and fibroblast growth factor 7 (FGF7), which have been reported in recent years as growth factors involved in hair growth, and aims to provide a composition that promotes the production of these factors, promotes the thickening of hair, normalizes the hair cycle, and affords a good preventive or improving effect on thinning hair and hair loss.

### [Solution to Problem]

The present inventors have conducted intensive studies in an attempt to solve the above-mentioned problems, and found that each of tryptophan, glutamine, and citrulline can promote VEGF production in hair papilla cells, promote division and proliferation of hair matrix cells, suppress shortening of the anagen phase of hair, normalize the hair cycle by maintaining the anagen phase of hair, and promote thickening of hair by promoting proliferation of hair papilla cells.

In addition, they have found that ornithine has the ability to promote the FGF7 production in hair papilla cells, shorten the telogen phase which is from the degeneration of the hair root until a hair begins to grow anew, promote the transition from the telogen phase to the anagen phase to normalize the hair cycle, and promote the division and proliferation of hair matrix cells, thus promoting the new hair growth, and further that ornithine can promote proliferation of hair papilla cells to promote hair thickening.

Furthermore, by formulating a composition for promoting hair thickening, a composition for maintaining the anagen phase of the hair cycle, a composition for promoting VEGF production, a composition for promoting transition from the telogen phase to the anagen phase of the hair cycle, a composition for promoting FGF7 production, and a composition for promoting normalization of the hair cycle, each containing the above-mentioned amino acid, the above-mentioned problems have been solved.

That is, the present invention relates to the following.
[1] A composition for promoting hair thickening, comprising one or more members selected from the group consisting of citrulline, tryptophan, glutamine, and ornithine.
[2] The composition of [1], wherein a total content of one or more members selected from the group consisting of citrulline, tryptophan, glutamine, and ornithine is 0.1 µM to 1 M.
[3] The composition of [1] or [2], wherein the composition comprises one or more members selected from the group consisting of citrulline, tryptophan, and glutamine, and ornithine.
[4] The composition of [3], wherein a content ratio of one or more members selected from the group consisting of citrulline, tryptophan, and glutamine, and ornithine (one or more members selected from the group consisting of citrulline, tryptophan, and glutamine:ornithine) is 1:0.1 to 1:0.5 in molar ratio.
[5] A composition for maintaining the anagen phase of the hair cycle, comprising one or more members selected from the group consisting of citrulline, tryptophan, and glutamine.
[6] The composition of [5], wherein a total content of one or more members selected from the group consisting of citrulline, tryptophan, and glutamine is 0.1 µM to 1 M.
[7] A composition for promoting VEGF production, comprising one or more members selected from the group consisting of citrulline, tryptophan, and glutamine.
[8] The composition of [7], wherein a total content of one or more members selected from the group consisting of citrulline, tryptophan, and glutamine is 0.1 µM to 1 M.
[9] A composition for promoting transition from the telogen phase to the anagen phase of the hair cycle, comprising ornithine.
[10] The composition of [9], wherein a content of the ornithine is 1 µM to 1 M.
[11] A composition for promoting FGF7 production, comprising ornithine.
[12] The composition of [11], wherein a content of the ornithine is 1 µM to 1 M.
[13] A composition for promoting normalization of the hair cycle, comprising one or more members selected from the group consisting of citrulline, tryptophan, glutamine, and ornithine.
[14] The composition of [13], wherein a total content of one or more members selected from the group consisting of citrulline, tryptophan, glutamine, and ornithine is 0.1 µM to 1 M.
[15] The composition of [13] or [14], wherein the composition comprises one or more members selected from the group consisting of citrulline, tryptophan, and glutamine, and ornithine.
[16] The composition of [15], wherein a content ratio of one or more members selected from the group consisting of citrulline, tryptophan, and glutamine, and ornithine (one or more members selected from the group consisting of citrulline, tryptophan, and glutamine:ornithine) is 1:0.1 to 1:0.5 in molar ratio.
[17] A food for preventing or improving thinning hair or hair loss, comprising either or both of the composition for promoting VEGF production of [7] or [8] and the composition for promoting FGF7 production of [11] or [12].
[18] An external composition for preventing or improving thinning hair or hair loss, comprising either or both of the composition for promoting VEGF production of [7] or [8] and the composition for promoting FGF7 production of [11] or [12].
[19] The external composition of [18], wherein the composition is a skin external quasi-drug.
[20] The external composition of [18], wherein the composition is a hair cosmetic or a scalp cosmetic.
[21] A composition for preventing or improving androgenetic alopecia, comprising the composition for maintaining the anagen phase of the hair cycle of [5] or [6].
[22] A composition for preventing or improving androgenetic alopecia, comprising the composition for promoting VEGF production of [7] or [8].
[23] A composition for preventing or improving female pattern alopecia, comprising the composition for promoting transition from the telogen phase to the anagen phase of the hair cycle of [9] or [10].
[24] A composition for preventing or improving female pattern alopecia, comprising the composition for promoting FGF7 production of [11] or [12].
[25] The composition of any of [1] to [16], wherein the composition is a composition for suppressing or ameliorating scalp itchiness.

### [Advantageous Effects of Invention]

According to the present invention, a composition for promoting hair thickening which can promote thickening of hair can be provided.

The composition for promoting hair thickening of the present invention can promote production of VEGF or FGF7 in hair papilla cells and proliferation of hair papilla cells, and grow long, thick, and healthy hair.

According to the present invention, a composition for maintaining the anagen phase of the hair cycle can be provided.

The composition for maintaining the anagen phase of the hair cycle of the present invention can suppress shortening of the anagen phase of hair and maintain the anagen phase of hair, by promoting VEGF production in hair papilla cells.

According to the present invention, a composition for promoting VEGF production which can promote VEGF production in hair papilla cells can be provided.

The composition for promoting VEGF production of the present invention can promote VEGF production in hair papilla cells, promote division and proliferation of hair matrix cells, suppress shortening of the anagen phase of hair, normalize the hair cycle by maintaining the anagen phase of hair, and promote thickening of hair by promoting proliferation of hair papilla cells.

Therefore, the composition for maintaining the anagen phase of the hair cycle and the composition for promoting VEGF production of the present invention can be preferably utilized, particularly as a hair-growing agent.

According to the present invention, a composition for promoting transition from the telogen phase to the anagen phase of the hair cycle can be provided.

The composition for promoting transition from the telogen phase to the anagen phase of the hair cycle of the present invention can shorten the telogen phase which is from the degeneration of the hair root until a hair begins to grow anew, and promote the transition from the telogen phase to the anagen phase of hair, by promoting FGF7 production in hair papilla cells.

According to the present invention, a composition for promoting FGF7 production which can promote FGF7 production in hair papilla cells can be provided.

The composition for promoting FGF7 production of the present invention can shorten the telogen phase which is from the degeneration of the hair root until a hair begins to grow anew by promoting FGF7 production in hair papilla cells, normalize the hair cycle by promoting the transition from the telogen phase to the anagen phase, and promote growing of a new hair by promoting the division and proliferation of hair matrix cells.

Therefore, the composition for promoting transition from the telogen phase to the anagen phase of the hair cycle and the composition for promoting FGF7 production of the present invention are preferably used, particularly as a new-hair-growing agent.

According to the present invention, a composition for promoting normalization of the hair cycle can be provided.

The composition for promoting the normalization of the hair cycle of the present invention can normalize the hair cycle by either or both of the action of maintaining the anagen phase of the hair cycle by promoting VEGF production, and the action of promoting transition from the telogen phase to the anagen phase of the hair cycle by promoting FGF7 production.

The composition for promoting hair thickening, the composition for maintaining the anagen phase of the hair cycle, and the composition for promoting VEGF production of the present invention, the composition for promoting transition from the telogen phase to the anagen phase of the hair cycle and the composition for promoting FGF7 production of the present invention, and the composition for promoting normalization of the hair cycle of the present invention can favorably improve or prevent thinning hair or hair loss, and particularly useful for androgenetic alopecia and female pattern alopecia.

Furthermore, the composition for promoting hair thickening, the composition for maintaining the anagen phase of the hair cycle, and the composition for promoting VEGF production of the present invention, the composition for promoting transition from the telogen phase to the anagen phase of the hair cycle and the composition for promoting FGF7 production of the present invention, and the composition for promoting normalization of the hair cycle of the present invention contain amino acids that have been eaten much as active ingredients, are highly safe, can be used not only for external use, but also for oral ingestion or oral administration, and can be used continuously for a long period of time.

### [Brief Description of Drawings]

[Fig. 1]

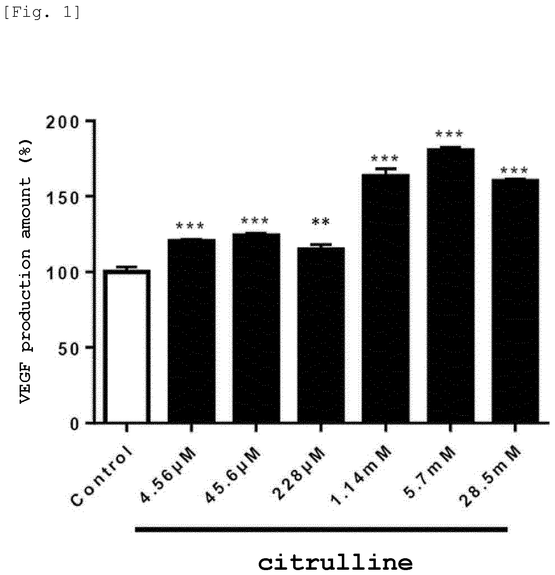

   Fig. 1 is a diagram showing the influence of citrulline on VEGF production in hair papilla cells in Experimental Example 1. In the Figure, "**" indicates a significant difference at P<0.01 from the control, and "***" indicates a significant difference at P<0.001 from the control.
[Fig. 2]

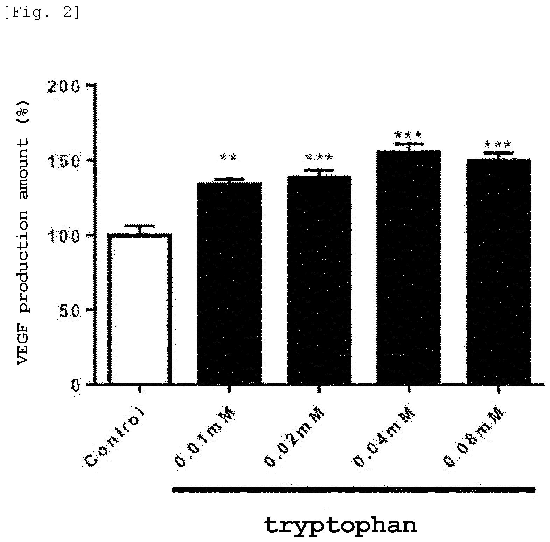

   Fig. 2 is a diagram showing the influence of tryptophan on VEGF production in hair papilla cells in Experimental Example 1. In the Figure, "**" indicates a significant difference at P<0.01 from the control, and "***" indicates a significant difference at P<0.001 from the control.
[Fig. 3]

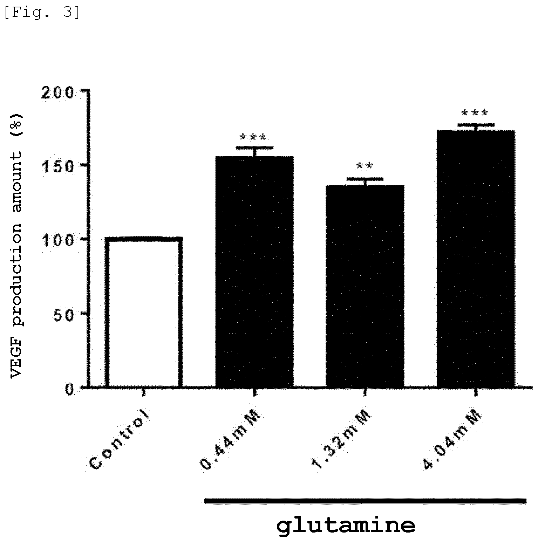

   Fig. 3 is a diagram showing the influence of glutamine on VEGF production in hair papilla cells in Experimental Example 1. In the Figure, "**" indicates a significant difference at P<0.01 from the control, and "***" indicates a significant difference at P<0.001 from the control.
[Fig. 4]

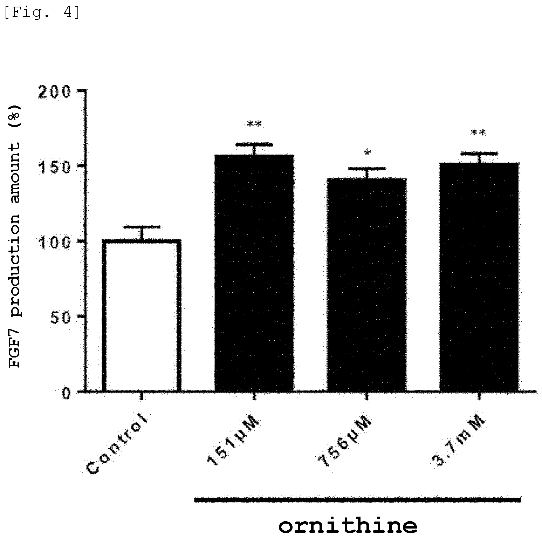

   Fig. 4 is a diagram showing the influence of ornithine on FGF7 production in hair papilla cells in Experimental Example 2. In the Figure, "*" indicates a significant difference at P<0.05 from the control, and "**" indicates a significant difference at P<0.01 from the control.
[Fig. 5]

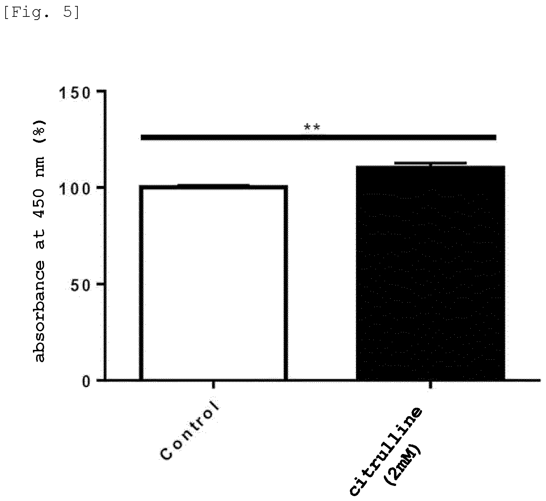

   Fig. 5 is a diagram showing the influence of citrulline on proliferation of hair papilla cells in Experimental Example 3. In the Figure, "**" indicates a significant difference at P<0.01 from the control.
[Fig. 6]

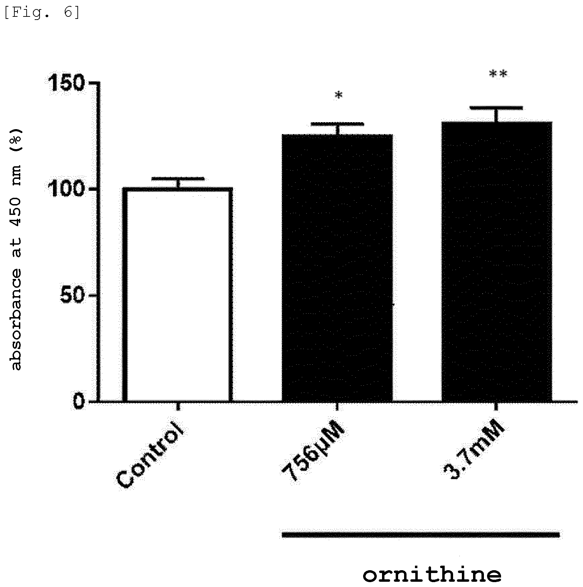

   Fig. 6 is a diagram showing the influence of ornithine on proliferation of hair papilla cells in Experimental Example 4. In the Figure, "*" indicates a significant difference at P<0.05 from the control, and "**" indicates a significant difference at P<0.01 from the control.
[Fig. 7]

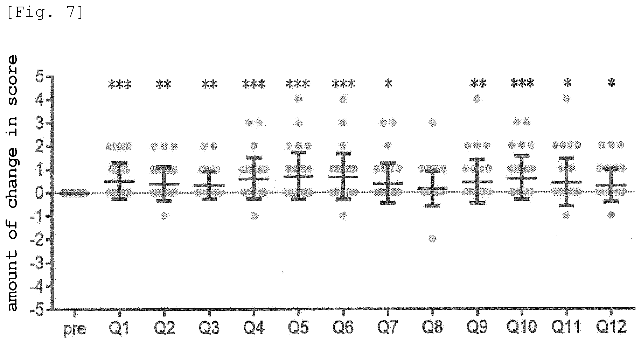

   Fig. 7 is a diagram showing the amount of change in score for each evaluation item regarding hair in Experimental Example 5 before and after ingestion of the test food. In the Figure, "*" indicates a significant difference at P<0.05 from before ingestion (pre), "**" indicates a significant difference at P<0.01 from before ingestion (pre), and "***" indicates a significant difference at P<0.001 from ingestion (pre).
[Fig. 8]

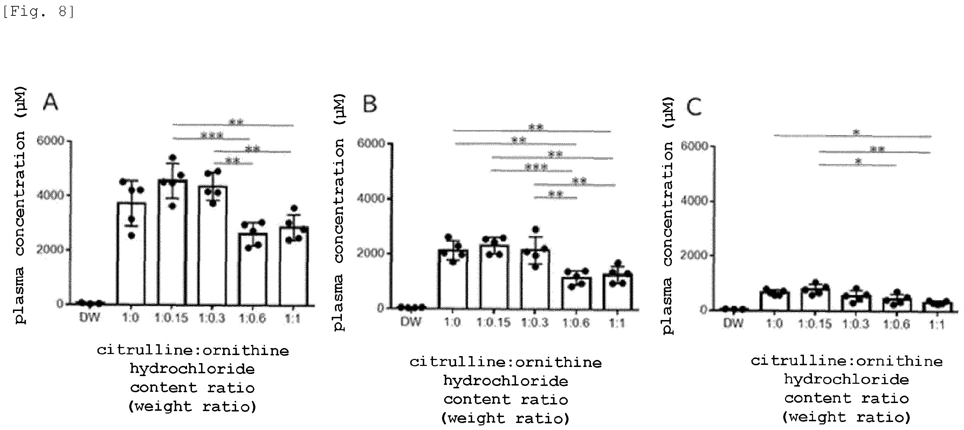

   Fig. 8 is a diagram showing the relationship between the content ratio of citrulline and ornithine hydrochloride, and the plasma concentration of citrulline in Experimental Example 6. In the Figure, A shows the results 30 min after administration of the test solution, B shows the results 1 hr after administration of the test solution, and C shows the results 2 hr after administration of the test solution. In the Figure, "DW" indicates a distilled water administration group. Each "*" indicates a significant difference at P<0.05, each "**" indicates a significant difference at P<0.01, and each "***" indicates a significant difference at P<0.001.
[Fig. 9]

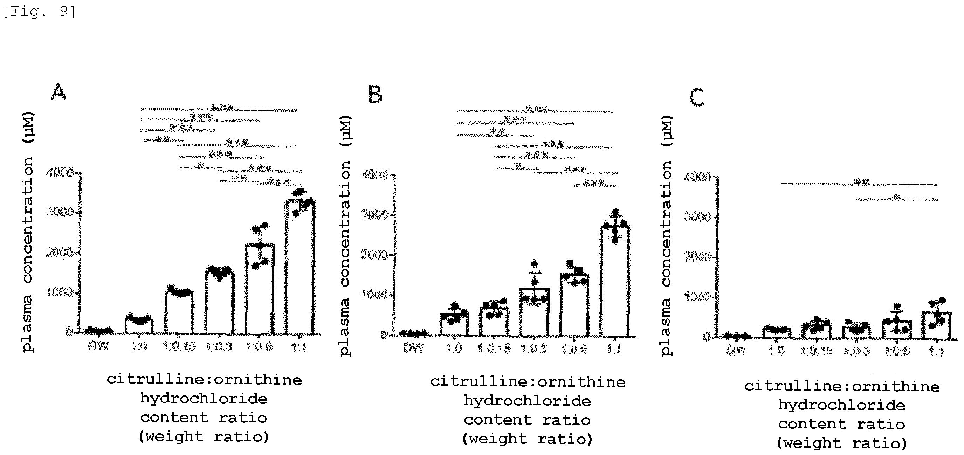

   Fig. 9 is a diagram showing the relationship between the content ratio of citrulline and ornithine hydrochloride, and the plasma concentration of ornithine in Experimental Example 6. In the Figure, A shows results 30 min after administration of the test solution, B shows results 1 hr after administration of the test solution, and C shows results 2 hr after administration of the test solution. In the Figure, "DW" indicates a distilled water administration group. Each "*" indicates a significant difference at P<0.05, each "**" indicates a significant difference at P<0.01, and each "***" indicates a significant difference at P<0.001.

### [Description of Embodiments]

The present invention provides a composition for promoting hair thickening.

The composition for promoting hair thickening of the present invention (hereinafter sometimes to be referred to as "the thickening promoting composition of the present invention" in the present specification) contains one or more member selected from the group consisting of citrulline, tryptophan, glutamine, and ornithine.

Citrulline, tryptophan, and glutamine each have actions of promoting proliferation of hair papilla cells and promoting hair thickening, via promotion of VEGF (vascular endothelium growth factor) production in hair papilla cells.

In addition, ornithine has actions of promoting FGF7 (fibroblast growth factor 7) production in hair papilla cells and proliferation of hair papilla cells, and promoting hair thickening.

Citrulline contained as an active ingredient in the thickening promoting composition of the present invention has the general chemical name of 2-amino-5-(carbamoylamino)pentanoic acid, and is an amino acid constituting the urea cycle.

Tryptophan contained as an active ingredient in the thickening promoting composition of the present invention has the general chemical name of 2-amino-3-(indolyl)propionic acid, and is one of the aromatic amino acids.

Glutamine contained as an active ingredient in the thickening promoting composition of the present invention has the general chemical name of 2-amino-4-carbamoylbutanoic acid, and is an amino acid having an amide on the side chain.

Ornithine contained as an active ingredient in the thickening promoting composition of the present invention has the general chemical name of 2,5-diamino pentanoic acid, and is an amino acid constituting the urea cycle.

The citrulline, tryptophan, glutamine, and ornithine to be used may be any of L-form, D-form and DL-form. The L-form and DL-form are preferably used, and L-form is more preferably used.

In addition, the "citrulline", "tryptophan", "glutamine", and "ornithine" to be used may be not only a free form but also in a salt form. The terms "citrulline", "tryptophan", "glutamine", and "ornithine" in the present specification are each a concept encompassing even a salt. The salt form is not particularly limited as long as it is a pharmacologically acceptable salt, and acid addition salt, salt with base and the like can be mentioned.

Specifically, salts with inorganic bases, salts with organic bases, salts with inorganic acids, salts with organic acids, salts with amino acid and the like can be mentioned.

Examples of the salts with inorganic bases include salts with alkali metals such as lithium, sodium, potassium and the like, salts with alkaline earth metals such as magnesium, calcium and the like, ammonium salt and the like.

Examples of the salts with organic bases include salts with alkanolamine such as monoethanolamine, diethanolamine, triethanolamine and the like, salts with heterocyclic amine such as morpholine, piperidine and the like, and the like.

Examples of the salts with inorganic acids include salts with hydrohalic acid (hydrochloric acid, hydrobromic acid, hydroiodic acid, etc.), sulfuric acid, nitric acid, phosphoric acid and the like, and the like.

Examples of the salts with organic acids include salts with monocarboxylic acid such as formic acid, acetic acid, propanoic acid and the like; salts with saturated dicarboxylic acid such as oxalic acid, malonic acid, malic acid, succinic acid and the like; salts with unsaturated dicarboxylic acid such as maleic acid, fumaric acid and the like; salts with tricarboxylic acid such as citric acid and the like; salts with keto acid such as α-ketoglutaric acid and the like, and the like.

Examples of the salts with amino acid include salts with aliphatic amino acid such as alanine and the like; salts with aromatic amino acid such as tyrosine and the like; salts with basic amino acid such as arginine and the like; salts with acidic amino acid such as aspartic acid, glutamic acid and the like; salts with amino acid forming lactam such as pyroglutamic acid and the like; and the like.

The above-mentioned salts may each be a hydrate (hydrate salt), and examples of the hydrate include 1 hydrate to 6 hydrate and the like.

In the present invention, one member of "citrulline", "tryptophan", "glutamine", and "ornithine" in the above-mentioned free form or a salt form may be used singly, or two or more members thereof may be used in combination.

Each of "citrulline", "tryptophan", "glutamine", and "ornithine" in a free form, hydrochloride thereof, and the like are preferably used for the purpose of the present invention.

In the present invention, "citrulline", "tryptophan", "glutamine", and "ornithine" in a free form or in the form of a salt to be used may be extracted from animals, plants or the like, which are naturally present, and purified, or obtained by a chemical synthesis method, a fermentation method, an enzyme method or a gene recombinant method. Commercially available products provided by each company may also be utilized.

In the thickening promoting composition of the present invention, one member or two or more members selected from the group consisting of citrulline, tryptophan, glutamine, and ornithine can be used.

When the thickening promoting composition of the present invention is provided in a solution form, the total content of one or more members selected from the group consisting of citrulline, tryptophan, glutamine, and ornithine in the thickening promoting composition of the present invention and the like is generally 0.1 µM to 1 M, preferably 0.3 µM to 500 mM, more preferably 1 µM to 100 mM.

In the present specification, referring to the "total amount of these" or the "total amount of one or more members selected from the group consisting of citrulline, tryptophan, glutamine, and ornithine" or the "total amount of one or more members selected from the group consisting of citrulline, tryptophan, and glutamine", when one selected from the group consisting of citrulline, tryptophan, glutamine, and ornithine, or the group consisting of citrulline, tryptophan, and glutamine is contained or used, it means the content or amount of use of the amino acid; and when two or more are selected and contained or used, it means a total of the contents or amount of use thereof.

In the present specification, referring to the "total content", when one is selected from the group consisting of citrulline, tryptophan, glutamine, and ornithine, or the group consisting of citrulline, tryptophan, and glutamine, and contained, it means the content of the amino acid, and when two or more are selected and contained, it means a total amount of the contents thereof.

From the aspect of hair thickening promoting effect, the thickening promoting composition of the present invention preferably contains one or more members selected from the group consisting of citrulline, tryptophan, and glutamine, and ornithine, more preferably citrulline and ornithine.

The content ratio of one or more members selected from the group consisting of citrulline, tryptophan, and glutamine, and ornithine (one or more members selected from the group consisting of citrulline, tryptophan, and glutamine:ornithine) is preferably 1:0.1 to 1:0.5, more preferably 1:0.15 to 1:0.32, in molar ratio.

The present invention provides a composition for maintaining the anagen phase of the hair cycle based on the promotion of VEGF production. In addition, the present invention provides a composition for promoting VEGF production.

VEGF (vascular endothelium growth factor) is a growth factor that is produced by hair papilla cells, promotes division and proliferation of hair matrix cells, is involved in maintaining the anagen phase of hair, and has an action of promoting proliferation of hair papilla cells.

The composition for maintaining the anagen phase of the hair cycle of the present invention (hereinafter sometimes to be referred to as "the composition for maintaining the anagen phase of the present invention" in the present specification) and the composition for promoting VEGF production of the present invention each contain one member or two or more members selected from the group consisting of citrulline, tryptophan, and glutamine.

Citrulline, tryptophan, and glutamine contained as active ingredients in each of the composition for maintaining the anagen phase and the composition for promoting VEGF production of the present invention are as described above in the thickening promoting composition of the present invention.

When the composition for maintaining the anagen phase and the composition for promoting VEGF production of the present invention are provided in the form of solutions, the total content of one or more members selected from the group consisting of citrulline, tryptophan, and glutamine is generally 0.1 µM to 1 M, preferably 0.3 µM to 500 mM, more preferably 1 µM to 100 mM.

The present invention also provides a composition for promoting transition from the telogen phase to the anagen phase of the hair cycle based on the promotion of VEGF production. Furthermore, the present invention provides a composition for promoting FGF7 production.

FGF7 (fibroblast growth factor 7) is a growth factor that is produced by hair papilla cells, is involved in the transition from the telogen phase to the anagen phase of hair, and has an action of promoting division and proliferation of hair matrix cells.

The composition for promoting transition from the telogen phase to the anagen phase of the hair cycle of the present invention (hereinafter sometimes to be referred to as "the composition for promoting the anagen phase transition of the present invention" in the present specification) and the composition for promoting FGF7 production of the present invention contain ornithine.

Ornithine contained as an active ingredient in each of the composition for promoting the anagen phase transition of the present invention and the composition for promoting FGF7 production of the present invention is as described above in the thickening promoting composition of the present invention.

When the composition for promoting the anagen phase transition and the composition for promoting FGF7 production of the present invention are provided in the form of solutions, the content of ornithine in each them is generally 1 µM to 1 M, preferably 5 µM to 500 mM, more preferably 10 µM to 100 mM.

Furthermore, the present invention provides a composition for promoting the normalization of the hair cycle that can normalize the hair cycle by either or both of the action of maintaining the anagen phase of the hair cycle by promoting VEGF production, and the action of promoting transition from the telogen phase to the anagen phase of the hair cycle by promoting FGF7 production.

The composition for promoting the normalization of the hair cycle of the present invention contains one member or two or more members selected from the group consisting of citrulline, tryptophan, glutamine, and ornithine.

Citrulline, tryptophan, glutamine, and ornithine contained as active ingredients in the composition for promoting the normalization of the hair cycle of the present invention are as described above in the thickening promoting composition of the present invention.

When the composition for promoting the normalization of the hair cycle of the present invention is provided in the form of a solution, the total content of one or more members selected from the group consisting of citrulline, tryptophan, glutamine, and ornithine is generally 0.1 µM to 1 M, preferably 0.3 µM to 500 mM, more preferably 1 µM to 100 mM.

From the aspect of hair cycle normalization promoting effect, the composition for promoting the normalization of the hair cycle of the present invention preferably contains one or more members selected from the group consisting of citrulline, tryptophan, and glutamine, and ornithine, more preferably citrulline and ornithine.

The content ratio of one or more members selected from the group consisting of citrulline, tryptophan, and glutamine, and ornithine (one or more members selected from the group consisting of citrulline, tryptophan, and glutamine:ornithine) is preferably 1:0.1 to 1:0.5, more preferably 1:0.15 to 1:0.32, in molar ratio.

The thickening promoting composition of the above-mentioned present invention, the composition for maintaining the anagen phase and the composition for promoting VEGF production of the present invention, the composition for promoting the anagen phase transition and the composition for promoting FGF7 production of the present invention, and the composition for promoting the normalization of the hair cycle of the present invention can be respectively formulated as oral compositions in the forms of solid form (e.g., tablet, coated tablet, chewable tablet, pill, (micro)capsule, granule, fine granule, powder, and the like); liquid form (e.g., elixir, lemonade, syrup, suspension, emulsion, and the like); semisolid form (e.g., jelly for oral administration and the like); and the like, skin external preparations or skin external quasi-drugs such as external liquid (e.g., liniment, lotion, and the like); spray (e.g., external aerosol, pump spray, and the like); ointment (e.g., oleagenous ointment, water-soluble ointment, and the like); cream (e.g., oil-in-water type cream, water-in-oil type cream, and the like); gel (e.g., aqueous gel, oily gel, and the like); and the like, external compositions such as hair cosmetics or cosmetics for scalp (e.g., liquid, milky liquid, spray, paste, cream, gel, and the like) and the like.

Each composition of the present invention in the above-mentioned form can be prepared by a formulating means well known in the field of preparations, for example, the methods described in the Japanese Pharmacopoeia, seventeenth Edition, General Rules for preparation, [3] Monographs for Preparations.

In this case, various pharmacologically acceptable additives for preparations can be blended as necessary. The additive can be appropriately selected according to the form of each composition of the present invention mentioned above. For example, excipient, binder, disintegrant, lubricant, coating agent, base, solvent, diluent, solubilizing agent, solubilizer, emulsifier, dispersing agent, suspending agent, stabilizer, thickener, gelling agent, pH adjuster, antioxidant, antiseptic, preservative, corrigent, flavoring agent, sweetening agent, flavor, colorant, and the like can be mentioned.

Specifically, examples of the excipient include magnesium carbonate, titanium dioxide, saccharides (lactose, etc.), sugar alcohol (mannitol, etc.), casein, and the like.

Examples of the binder include gelatin, starch, cellulose and a derivative thereof, and the like.

Examples of the disintegrant include crospovidone, crystalline cellulose, and the like.

Examples of the lubricant include talc, magnesium stearate, and the like.

Examples of the coating agent include
methylmethacrylate·butylmethacrylate·dimethylaminoethylmethacry late copolymer,
ethylacrylate·methylmethacrylate·trimethylammmonioethylmethacry late chloride copolymer, and the like.

Examples of the base include animal oil, vegetable oil, hydrocarbon oil (liquid paraffin, etc.), polyethylene glycol, and the like.

Examples of the solvent include purified water, monovalent alcohol (ethanol, etc.), polyhydric alcohol (glycerol, etc.), and the like.

Examples of the emulsifier or dispersing agent include sorbitan fatty acid ester, glycerine fatty acid ester, polyoxyethylene sorbitan fatty acid ester, sucrose fatty acid ester, and the like.

Examples of the stabilizer include adipic acid, β-cyclodextrin, and the like.

Examples of the thickener include water-soluble polymer (sodium polyacrylate, carboxyvinyl polymer, etc.), polysaccharides (sodium alginate, xanthan gum, tragacanth, etc.), and the like.

Examples of the gelling agent include carboxyvinyl polymer, dextrin palmitate, and the like.

Examples of the pH adjuster include hydrochloric acid, sulfuric acid, acetic acid, citric acid, lactic acid, sodium hydroxide, potassium hydroxide, and the like.

Examples of the antioxidant include dibutylhydroxytoluene (BHT), butylhydroxyanisole (BHA), α-tocopherol, erythorbic acid, and the like.

Examples of the antiseptic or preservative include paraben (methylparaben, etc.), benzyl alcohol, sodium dehydroacetate, sorbic acid, and the like.

Examples of the corrigent or flavoring agent include ascorbic acid, erythritol, sodium L-glutamate, and the like.

Examples of the sweetening agent include aspartame, licorice extract, saccharin, and the like.

Examples of the flavor include l-menthol, d-camphor, cineol, and the like.

Examples of the colorant include tar pigment (red No. 2, blue No. 1, yellow No. 4, etc.), inorganic pigment (red iron oxide, yellow iron oxide, black iron oxide, etc.), natural dye (annatto dye, turmeric dye, β-carotene, etc.), and the like.

The amount of use of each of the above-mentioned compositions of the present invention is appropriately determined according to the condition or degree of thinning hair or hair loss, gender, age, body weight of the subject to whom the composition is applied (hereinafter also to be referred to as the "application subject" in the present specification), the form of the composition, use method, and the like.

As used herein, the "amount to be used" refers to an amount of ingestion or dose in the case of oral composition, and an amount to be applied in the case of external composition.

When the application subject is a human adult, the amount of use of each of the thickening promoting composition of the present invention and the composition for promoting normalization of the hair cycle of the present invention is generally 0.01 mg/kg body weight to 100 g/kg body weight, preferably 0.1 mg/kg body weight to 50 g/kg body weight, more preferably 1 mg/kg body weight to 10 g/kg body weight, per day, as a total amount of one or more members selected from the group consisting of citrulline, tryptophan, glutamine, and ornithine (when contained in a salt form, an amount converted to a free form), for ingestion or administration as an oral composition.

When it is applied as an external composition, the amount is generally 0.15 mg/cm² to 150 mg/cm², preferably 0.75 mg/cm² to 75 mg/cm², more preferably 1.5 mg/cm² to 15 mg/cm², per application.

When the application subject is a human adult, the amount of use of each of the composition for maintaining the anagen phase and the composition for promoting VEGF production of the present invention is generally 0.01 mg/kg body weight to 100 g/kg body weight, preferably 0.1 mg/kg body weight to 50 g/kg body weight, more preferably 1 mg/kg body weight to 10 g/kg body weight, per day, as a total amount of one or more members selected from the group consisting of citrulline, tryptophan, and glutamine (when contained in a salt form, an amount converted to a free form), for ingestion or administration as an oral composition.

When it is applied as an external composition, the amount is generally 0.15 mg/cm² to 150 mg/cm², preferably 0.75 mg/cm² to 75 mg/cm², more preferably 1.5 mg/cm² to 15 mg/cm², per application.

When the application subject is a human adult, the amount of use of each of the composition for promoting the anagen phase transition and the composition for promoting FGF7 production of the present invention is generally 0.01 mg/kg body weight to 100 g/kg body weight, preferably 0.1 mg/kg body weight to 50 g/kg body weight, more preferably 1 mg/kg body weight to 10 g/kg body weight, per day, as an amount of ornithine (when contained in a salt form, an amount converted to a free form), for ingestion or administration as an oral composition.

When it is applied as an external composition, the amount is generally 0.15 mg/cm² to 150 mg/cm², preferably 0.75 mg/cm² to 75 mg/cm², more preferably 1.5 mg/cm² to 15 mg/cm², per application.

The above-mentioned amount of ingestion or dose per day may be ingested or administered at one time, or in two or more divided portions (e.g., 2 to 5 portions).

The timing of use of each of the above-mentioned compositions of the present invention is not particularly limited. When ingested or administered as an oral composition, it may be ingested or administered before or after meal, or together with meal. When applied as an external composition, it can be appropriately used, for example, after washing face in the morning, after taking bath, and the like.

While the number of use of each of the above-mentioned compositions of the present invention is not particularly limited, since the expected hair thickening promoting effect and the like can be obtained, multiple times of continuous use by the application subject is preferred.

Since citrulline, tryptophan, glutamine, and ornithine contained in each of the above-mentioned compositions of the present invention are all amino acids that have been eaten much and have high safety, each of the above-mentioned compositions of the present invention can be continuously used by the application subject. In order to effectively prevent or improve thinning hair or hair loss, use for a long period of time (e.g., 2 weeks or more) is preferred.

When each composition of the present invention mentioned above is provided as an oral composition, it can be formulated as a unit package form. In the present specification, the term "unit package form" means a form of one or more units with a particular amount (e.g., intake per one time, etc.) as one unit is/are packed in one container or package. For example, a unit package form with intake per one time as one unit is referred to as "unit package form for intake per one time". A container or package used for the unit package form can be appropriately selected according to the form, and the like of each composition of the present invention mentioned above. For example, paper container or bag, plastic container or bag, pouch, aluminum can, steel can, glass bottle, pet bottle, PTP (press through pack) package sheet, and the like can be mentioned.

In addition, when each composition of the present invention mentioned above is provided in the form of an external composition, the amount for single application can be discharged by a spray using an aerosol can or a pump container having a constant amount discharge mechanism.

The application subject of each composition of the present invention mentioned above includes mammals (human, mouse, rat, hamster, rabbit, cat, dog, bovine, horse, donkey, swine, sheep, monkey, etc.). When each composition of the present invention mentioned above is applied to an application subject animal other than human (hereinafter to be also simply referred to as "target animal"), each amount to be use thereof can be appropriately set according to the kind, sex, body weight, and the like of the target animal.

The composition for promoting hair thickening of the present invention can promote production of VEGF or FGF7 in hair papilla cells and proliferation of hair papilla cells, and grow long, thick, and healthy hair.

The composition for maintaining the anagen phase and the composition for promoting VEGF production of the present invention promote VEGF production in hair papilla cells and promote division and proliferation of hair matrix cells. They can correct the shortened anagen phase of hair, normalize the hair cycle by maintaining a normal period of the anagen phase, and promote hair length growth in those with thinning hair or hair loss.

The composition for promoting the anagen phase transition and the composition for promoting FGF7 production of the present invention promote FGF7 production in hair papilla cells. They can normalize the hair cycle by promoting transition from the telogen phase to the anagen phase after degeneration of the hair root, and promote new hair growth by promoting the division and proliferation of hair matrix cells in those with thinning hair or hair loss.

Furthermore, the composition for promoting the normalization of the hair cycle of the present invention can normalize the hair cycle by either or both of the action of maintaining the anagen phase of the hair cycle by promoting VEGF production, and the action of promoting transition from the telogen phase to the anagen phase of the hair cycle by promoting FGF7 production.

Therefore, each of the above-mentioned compositions of the present invention can improve thinning hair or hair loss in those with thinning hair or hair loss such as androgenetic alopecia, female pattern alopecia, and the like, and can prevent thinning hair or hair loss in middle-aged and elderly persons at a risk of development or progression of thinning hair or hair loss, and the like.

The composition for maintaining the anagen phase and the composition for promoting VEGF production of the present invention are particularly effective for preventing or improving androgenetic alopecia.

In addition, the composition for promoting the anagen phase transition and the composition for promoting FGF7 production of the present invention is particularly effective for preventing or improving female pattern alopecia.

Furthermore, each composition of the present invention mentioned above has an action of suppressing or ameliorating itchiness of the scalp, in addition to the above-mentioned hair growth-promoting, hair cycle-normalizing, and new-hair-growth promoting actions.

Hence, each composition of the present invention mentioned above can also be used as a composition for suppressing or ameliorating itchiness of the scalp.

The present invention also provides a hair-growing agent containing the thickening promoting composition, the composition for maintaining the anagen phase, or the composition for promoting VEGF production of the present invention.

As used herein, the "hair growing" means to promote hair growth, and in the present invention, it means to lengthen hair and promote the proliferation of hair papilla cells to make the hair thick and elastic.

The hair-growing agent of the present invention can be provided in the form of an oral preparation or a skin external preparation according to the form of the thickening promoting composition of the present invention and the like to be contained therein.

The content of the thickening promoting composition of the present invention in the hair-growing agent of the present invention is generally 0.000005 wt% to 99 wt%, preferably 0.00001 wt% to 95 wt%, more preferably 0.00005 wt% to 90 wt%, as a total content of one or more members selected from the group consisting of citrulline, tryptophan, glutamine, and ornithine (when contained in a salt form, calculated as an amount converted to a free form).

The content of the composition for maintaining the anagen phase or the composition for promoting VEGF production of the present invention in the hair-growing agent of the present invention is generally 0.000005 wt% to 99 wt%, preferably 0.00001 wt% to 95 wt%, more preferably 0.00005 wt% to 90 wt%, as a total content of one or more members selected from the group consisting of citrulline, tryptophan, and glutamine (when contained in a salt form, calculated as an amount converted to a free form).

The amount of use (amount of ingestion or dose in the case of oral preparation form, amount of application in the case of skin external preparation form) of the hair-growing agent of the present invention can be appropriately determined according to the condition and degree of thinning hair or hair loss of the subject for whom the hair-growing agent of the present invention is used, the age, gender, and body weight of the subject, form of the hair-growing agent of the present invention, and the like.

Specifically, the amount of use of one or more members selected from the group consisting of citrulline, tryptophan, glutamine, and ornithine can be determined such that it is the amount of use per day mentioned above for the thickening promoting composition of the present invention. In addition, the amount of use of one or more members selected from the group consisting of citrulline, tryptophan, and glutamine can be determined such that it is the amount of use per day mentioned above for each of the composition for maintaining the anagen phase and the composition for promoting VEGF production of the present invention.

The present invention further provides a new-hair-growing agent containing the composition for promoting the anagen phase transition or the composition for promoting FGF7 production of the present invention.

As used herein, the "new-hair-growing" refers to the growth of hair from a state in which hair does not exist in hair follicles.

The new-hair-growing agent of the present invention can be provided in the form of an oral preparation or a skin external preparation according to the form of the composition for promoting the anagen phase transition of the present invention and the like contained therein.

The content of the composition for promoting the anagen phase transition or the composition for promoting FGF7 production of the present invention in the new-hair-growing agent of the present invention is generally 0.0001 wt% to 90 wt%, preferably 0.0005 wt% to 85 wt%, more preferably 0.001 wt% to 80 wt%, as the content of ornithine (when contained in a salt form, calculated as an amount converted to a free form).

The amount of use (amount of ingestion or dose in the case of oral preparation form, amount of application in the case of skin external preparation form) of the new-hair-growing agent of the present invention can be appropriately determined according to the condition and degree of thinning hair or hair loss of the subject for whom the new-hair-growing agent of the present invention is used, the age, gender, and body weight of the subject, form of the new-hair-growing agent of the present invention, and the like. It can be determined such that the amount of use of ornithine is the amount of use per day mentioned above for the composition for promoting the anagen phase transition and the composition for promoting FGF7 production of the present invention.

The hair-growing agent and the new-hair-growing agent of the present invention may also be used in combination with other hair-growing components and new-hair-growing components. Specific examples of such hair-growing components and new-hair-growing components include blood circulation promoters such as carpronium chloride, swertianin, and the like; hair matrix cell stimulants such as trans-3,4'-dimethyl-3-hydroxyflavanone (t-flavanone), pantothenylethylether, and the like; new-hair-growth promoters such as adenosine, cytopurine (6-benzylaminopurine), pentadecanoic acid glycerides, and the like; and the like.

The hair-growing agent of the present invention can be prepared by adding a general formulation additive to the thickening promoting composition of the present invention, the composition for maintaining the anagen phase or the composition for promoting VEGF production of the present invention as necessary, and according to a formulating means well known in the field of preparations, for example, the methods described in the Japanese Pharmacopoeia, seventeenth Edition, General Rules for preparation, [3] Monographs for Preparations and the like.

The new-hair-growing agent of the present invention can be prepared by adding a general formulation additive to the composition for promoting the anagen phase transition or the composition for promoting FGF7 production of the present invention as necessary, and in the same manner as described above.

The timing of use, the number of use, and the use period of the hair-growing agent and the new-hair-growing agent of the present invention can be set in the same manner as the timing, number, and period described above for the thickening promoting composition of the present invention and the like.

The hair-growing agent and the new-hair-growing agent of the present invention are preferably applied to those with thinning hair or hair loss such as androgenetic alopecia patients, female pattern alopecia patients, and the like, middle-aged and elderly persons at a risk of development or progression of thinning hair or hair loss, and the like. They can improve or prevent thinning hair or hair loss.

The present invention also provides a medicament for the prophylaxis or improvement of thinning hair or hair loss (hereinafter also to be referred to as "the medicament of the present invention" in the present specification).

The medicament of the present invention contains either or both of the above-mentioned composition for promoting VEGF production and the composition for promoting FGF7 production of the present invention.

Either or both of the composition for promoting VEGF production and the composition for promoting FGF7 production of the present invention can be directly used to give the medicament of the present invention, or blended with the above-mentioned formulation additives such as excipient, solvent, diluent, and the like to give the medicament of the present invention.

The content of the composition for promoting VEGF production of the present invention in the medicament of the present invention is generally 0.000005 wt% to 99 wt%, preferably 0.00001 wt% to 95 wt%, more preferably 0.00005 wt% to 90 wt%, as the total content of one or more members selected from the group consisting of citrulline, tryptophan, and glutamine (when contained in a salt form, calculated as an amount converted to a free form).

The content of the composition for promoting FGF7 production of the present invention in the medicament of the present invention is generally 0.0001 wt% to 90 wt%, preferably 0.0005 wt% to 85 wt%, more preferably 0.001 wt% to 80 wt%, as the content of ornithine (when contained in a salt form, as an amount converted to a free form).

From the aspects of the prophylactic or improving effect on thinning hair or hair loss, the medicament of the present invention preferably contains both the composition for promoting VEGF production and the composition for promoting FGF7 production of the present invention.

The composition for promoting VEGF production and the composition for promoting FGF7 production of the present invention are preferably contained in the medicament of the present invention such that the content ratio of one or more members selected from the group consisting of citrulline, tryptophan, and glutamine, and ornithine (one or more members selected from the group consisting of citrulline, tryptophan, and glutamine:ornithine) is 1:0.1 to 1:0.5, more preferably 1:0.15 to 1:0.32.

The amount of use (dose in the case of oral preparation form, amount of application in the case of skin external preparation form) of the medicament of the present invention can be appropriately determined according to the condition and degree of thinning hair or hair loss of the patients for whom the medicament of the present invention is used, the age, gender, and body weight of the patients, medicament form, and the like.

Specifically, it can be determined such that the amount of use of one or more members selected from the group consisting of citrulline, tryptophan, and glutamine is the amount of use per day mentioned above for the composition for maintaining the anagen phase and the composition for promoting VEGF production of the present invention.

In addition, it can be determined such that the amount of use of ornithine is the amount of use per day mentioned above for the composition for promoting the anagen phase transition and the composition for promoting FGF7 production of the present invention.

The medicament of the present invention can be provided in the form of an oral preparation or a skin external preparation, and can be produced by a means of formulation well known in the field of pharmaceutical preparations, such as the method described in the Japanese Pharmacopoeia, seventeenth Edition, General Rules for Preparation, [3] Monographs for Preparations, and the like.

The timing of use, the number of use, and the use period of the medicament of the present invention can be set in the same manner as the timing, number, and period described above for the composition for maintaining the anagen phase of the present invention, the composition for promoting the anagen phase transition of the present invention, and the like.

The medicament of the present invention can be preferably used by those with thinning hair or hair loss such as androgenetic alopecia patients, female pattern alopecia patients, and the like, middle-aged and elderly persons at a risk of development or progression of thinning hair or hair loss, and the like.

Furthermore, in the present invention, either or both of the composition for promoting VEGF production and the composition for promoting FGF7 production of the present invention provided in the form of oral compositions can be used by adding same to various foods. The food to which either or both of the composition for promoting VEGF production and the composition for promoting FGF7 production of the present invention is/are added is not particularly limited, and may be any as long as it is a food in the form generally served for meals or desserts. For example, either or both of the composition for promoting VEGF production and the composition for promoting FGF7 production of the present invention is/are added to drinks, and a suitable flavor is added when desired, whereby a drink (e.g., beverage etc.) can be provided. More specifically, either or both of the composition for promoting VEGF production and the composition for promoting FGF7 production of the present invention can be added to, for example, juice, milk, confectionery, jelly, yogurt, candy, and the like.

Either or both of the composition for promoting VEGF production and the composition for promoting FGF7 production of the present invention may be added to a food in an amount for ingestion per day such that the ingestion amount of one or more members selected from the group consisting of citrulline, tryptophan, and glutamine, or the ingestion amount of ornithine is the above-mentioned daily ingestion amount determined for each of the composition for maintaining the anagen phase and the composition for promoting VEGF production of the present invention, and the composition for promoting the anagen phase transition and the composition for promoting FGF7 production.

The present invention also provides a food for the prophylaxis or improvement of thinning hair or hair loss (hereinafter also to be referred to as "the food of the present invention").

The food of the present invention contains either or both of the composition for promoting VEGF production and the composition for promoting FGF7 production of the present invention provided as the above-mentioned oral composition.

The food of the present invention contains either or both of the composition for promoting VEGF production and the composition for promoting FGF7 production of the present invention and, where necessary, food additives such as production agent, thickening stabilizer, gum base, emulsifier, preservative, antioxidant, gloss agent, pH adjuster, sweetener, bitter taste, acidulant, colorant, flavor, and the like. Alternatively, the food composition of the present invention can be provided in various forms containing either or both of the composition for promoting VEGF production and the composition for promoting FGF7 production of the present invention, and food or food starting materials, for example, drinks such as juice, beverage water, teas, and the like; milk products such as lactobacillus drinks, fermented milk, butter, cheese, yogurt, processing milk, defatted milk, and the like; meat products such as ham, sausage, hamburg steak, and the like; fish meat paste products such as boiled fish paste, tubelike fish sausage, *satsuma-age,* and the like; egg products such as rolled Japanese-style omelette, egg tofu, and the like; confectioneries such as cookie, jelly, chewing gum, candy, snack confectionery, frozen dessert, and the like; bread; noodles; pickles; smoked product; dried fish; food boiled down in soy; salt-preserved product; soups; seasonings, and may be provided as bottled food, canned food, retort pouch food. In addition, forms such as powder, granule, sheet, capsule, tablet, jelly, and the like can be provided.

The food of the present invention can be preferably ingested by those with thinning hair or hair loss such as androgenetic alopecia patients, female pattern alopecia patients, and the like, middle-aged and elderly persons at a risk of development or progression of thinning hair or hair loss, and the like.

Therefore, the food of the present invention can also be provided as a food with health claims such as food for specified health uses for the prophylaxis or improvement of thinning hair or hair loss, a food with nutrient function claims, a food with functional claims attached with functional indications such as "promotion of hair growth", "alleviation of fallen hair", "reduction of fallen hair", "hair thickening", "alleviation of hair distress ", "suppression of scalp itchiness", and the like, a food for special dietary uses such as food for middle-aged and elderly persons, and the like, health supplement, and the like.

Furthermore, either or both of the composition for promoting VEGF production and the composition for promoting FGF7 production of the present invention provided as oral compositions can be used by adding to a food supplement.

The "food supplement" in the present invention refers to one ingested to aid nutrition other than one ingested as a food, and also includes nutritional supplement, supplement, and the like. When either or both of the composition for promoting VEGF production and a composition for promoting FGF7 production of the present invention is/are added to a food supplement, it/they can be prepared in a form such as tablet, capsule, powder, granule, suspension, chewable, syrup, and the like by adding other nutrition components and additives when desired.

The above-mentioned food of the present invention can be processed and produced by adding food additive as necessary to either or both of the composition for promoting VEGF production and the composition for promoting FGF7 production of the present invention or adding either or both of the composition for promoting VEGF production and the composition for promoting FGF7 production of the present invention to a food or food starting materials, and applying a general food production method.

The content of the composition for promoting VEGF production of the present invention in the food of the present invention can be appropriately determined according to the kind or form of the food, the level of thinning hair or hair loss-improving effect expected by the ingestion of the food, and the like. The total content of one or more members selected from the group consisting of citrulline, tryptophan, and glutamine (when contained in a salt form, calculated as an amount converted to a free form) is generally 0.000005 wt% to 99 wt%, preferably 0.00001 wt% to 95 wt%, more preferably 0.00005 wt% to 90 wt%, of the total weight of the food.

The content of the composition for promoting FGF7 production of the present invention in the food of the present invention in the content of ornithine (when contained in a salt form, as an amount converted to a free form) is generally 0.0001 wt% to 90 wt%, preferably 0.0005 wt% to 85 wt%, more preferably 0.001 wt% to 80 wt%.

From the aspects of the prophylactic or improving effect on thinning hair or hair loss, the food of the present invention preferably contains both the composition for promoting VEGF production and the composition for promoting FGF7 production of the present invention.

The composition for promoting VEGF production and the composition for promoting FGF7 production of the present invention are preferably contained in the food of the present invention such that the content ratio of one or more members selected from the group consisting of citrulline, tryptophan, and glutamine, and ornithine (one or more members selected from the group consisting of citrulline, tryptophan, and glutamine:ornithine) is 1:0.1 to 1:0.5, more preferably 1:0.15 to 1:0.32.

The daily ingestion amount of the food of the present invention can be set as an amount that can achieve ingestion of the aforementioned daily ingestion amount of one or more members selected from the group consisting of citrulline, tryptophan, and glutamine for the composition for maintaining the anagen phase and the composition for promoting VEGF production of the present invention, or as an amount that can achieve ingestion of the aforementioned daily ingestion amount of ornithine for the composition for promoting the anagen phase transition and the composition for promoting FGF7 production of the present invention.

The present invention also provides a commercial package containing either or both of the composition for promoting VEGF production and the composition for promoting FGF7 production of the present invention, and a written matter stating that either or both of the composition for promoting VEGF production and the composition for promoting FGF7 production of the present invention can or should be used for the prophylaxis or improvement of thinning hair or hair loss.

Furthermore, the present invention also provides a method for promoting VEGF or FGF7 production in a target animal in need of the promotion of VEGF or FGF7 production (hereinafter also to be referred to as "the method of the present invention" in the present specification).

The method of the present invention includes use (ingestion, administration, or application) of, in a target animal in need of the promotion of VEGF or FGF7 production, one or more members selected from the group consisting of citrulline, tryptophan, and glutamine in an amount effective for promoting the VEGF production in the target animal, or ornithine in an amount effective for promoting the FGF7 production.

Alternatively, the method of the present invention includes use of, in a target animal in need of the promotion of VEGF and FGF7 production, one or more members selected from the group consisting of citrulline, tryptophan, and glutamine, and ornithine, in an amount effective for promoting the VEGF and FGF7 production in the target animal.

As the target animal of the method of the present invention, human and mammals other than human such as mouse, rat, hamster, rabbit, cat, dog, bovine, horse, donkey, swine, sheep, monkey, and the like can be mentioned.

In the case of human, the method of the present invention is preferably applied to those with thinning hair or hair loss such as androgenetic alopecia patients, female pattern alopecia patients, and the like, middle-aged and elderly persons at a risk of development or progression of thinning hair or hair loss, and the like.

The amount of use of one or more members selected from the group consisting of citrulline, tryptophan, and glutamine , or the amount of use of ornithine in the method of the present invention, is appropriately determined according to the kind, age, gender, body weight, the condition of thinning hair or hair loss, degree, and the like of the target animal.

Specifically, the composition for maintaining the anagen phase and the composition for promoting VEGF production of the present invention can be used for a human or a target animal other than human in an amount similar to the above-mentioned amount of use and at the frequency and period mentioned above.

In addition, the composition for promoting the anagen phase transition and the composition for promoting FGF7 production of the present invention can be used for a human or a target animal other than human in an amount similar to the above-mentioned amount of use and at the frequency and period mentioned above.

Furthermore, the use method of one or more members selected from the group consisting of citrulline, tryptophan, and glutamine, or ornithine in the method of the present invention includes oral ingestion or oral administration, transdermal administration, and the like. These are preferred since they do not require the guidance and supervision of a doctor at a medical institution and can be performed conveniently.

### [Example]

The present invention is explained in more detail in the following by way of Experimental Examples.

### [Experimental Example 1] Study of influence of citrulline, tryptophan, and glutamine on VEGF production in hair papilla cells

Using a papilla cell growth medium (PCGM) (Toyobo Co., Ltd.), human hair papilla cells (Toyobo Co., Ltd.) were cultured for 4 days on a collagen-coated dish, and seeded on a collagen-coated 48 well plate at a cell density of 1.2×10⁴ cells/0.3 mL/well. The cells were cultured for one day in a carbon dioxide incubator (5 volume% carbon dioxide, 37°C), and used for the evaluation of the VEGF production amount.

For the evaluation of the VEGF production amount, 20 kinds of amino acids were mixed at the composition shown in Table 1, the components other than amino acids were composed as in Dulbecco's modified Eagle medium (DMEM), glucose was added at 1 g/L, and the resulting medium was used as an evaluation medium.

**[Table 1]**

| amino acid | concentration (mg/L) |
|---|---|
| glycine | 7.50 |
| L-alanine | 8.90 |
| L-serine | 10.50 |
| L-threonine | 23.75 |
| L-cystine dihydrochloride | 15.75 |
| L-methionine | 7.50 |
| L-glutamine | 146.00 |
| L-asparagine | 13.20 |
| L-glutamic acid | 14.70 |
| L-aspartic acid | 13.30 |
| L-valine | 23.50 |
| L-leucine | 26.25 |
| L-isoleucine | 26.25 |
| L-phenylalanine | 16.50 |
| L-tyrosine disodium dihydrate | 26.00 |
| L-tryptophan | 4.00 |
| L-lysine hydrochloride | 36.50 |
| L-arginine hydrochloride | 21.00 |
| L-histidine hydrochloride monohydrate | 10.50 |
| L-proline | 11.50 |

L-citrulline, L-tryptophan, and L-glutamine were respectively dissolved in the evaluation medium to prepare each solution.

In the above-mentioned hair papilla cell culture medium on the collagen-coated plate, the medium was replaced with the evaluation medium, the above-mentioned respective solutions of L-citrulline, L-tryptophan, and L-glutamine were added at a given concentration, and the cells were cultured for 3 days at 37°C, 5 volume% carbon dioxide (n=4 for L-citrulline, L-tryptophan, and L-glutamine at each addition concentration).

Then, the supernatant of the culture medium was collected, centrifuged at 1500 rpm, and the supernatant was collected and cryopreserved at -30°C.

The case in which similar culture and treatment were performed without adding each solution of L-citrulline, L-tryptophan, and L-glutamine was used as the control.

The concentration of VEGF in the supernatant of the culture was measured by thawing the collected supernatant and using the VEGF Enzyme-Linked ImmunoSorbent Assay (ELISA) kit (Abcam Plc.).

The measurement results were converted to relative values (%) with the VEGF concentration in the control as 100, and shown as mean±standard error in Figs. 1 to 3.

As shown in Figs. 1 to 3, a significant increase in the VEGF production amount was observed by adding each of L-citrulline, L-tryptophan, and L-glutamine as compared with the control.

In the case of L-citrulline, a significant increase in the VEGF production was observed by the addition at not less than 4.56 µM (Fig. 1, P<0.01 or P<0.001).

In the case of L-tryptophan, a significant increase in the VEGF production was observed by the addition at not less than 0.01 mM (10 µM) (Fig. 2, P<0.01 or P<0.001).

In the case of L-glutamine, a significant increase in the VEGF production was observed by the addition at not less than 0.44 mM (Fig. 3, P<0.01 or P<0.001).

It was confirmed from the above-mentioned results of Experimental Example 1 that each of citrulline, tryptophan, and glutamine has an action on hair papilla cells to promote the production of VEGF which is a growth factor involved in the control of the hair cycle.

Therefore, the possibility was suggested that citrulline, tryptophan, and glutamine each may promote VEGF production by hair papilla cells, normalize the hair cycle by maintaining a normal anagen phase of the hair cycle, and promote hair growth.

### [Experimental Example 2] Study of influence of ornithine on FGF7 production in hair papilla cells

In the same manner as in Experimental Example 1, human hair papilla cells were cultured for 4 days, and seeded on a collagen-coated 48 well plate at a cell density of 1.2×10⁴ cells/0.3 mL/well. The cells were cultured for one day in a carbon dioxide incubator (5 volume% carbon dioxide, 37°C), and used for the evaluation of the FGF7 production amount. For the evaluation of the FGF7 production amount, the same evaluation medium as in Experimental Example 1 was used.

L-ornithine was dissolved in the evaluation medium to prepare a solution.

In the above-mentioned hair papilla cell culture medium on the collagen-coated plate, the medium was replaced with the evaluation medium, the above-mentioned solution of L-ornithine was added at a given concentration, and the cells were cultured for 3 days at 37°C, 5 volume% carbon dioxide (n=3 at each addition concentration).

The case in which similar culture and treatment were performed without adding the solution of L-ornithine was used as the control.

Then, the supernatant of the culture medium was collected, centrifuged at 1500 rpm, and the supernatant was collected and cryopreserved at -30°C.

The concentration of FGF7 in the supernatant of the culture was measured by thawing the collected supernatant and using the FGF7 Enzyme-Linked ImmunoSorbent Assay (ELISA) kit (Abcam Plc.).

The measurement results were converted to relative values (%) with the FGF7 concentration in the control as 100, and shown as mean±standard error in Fig. 4.

As shown in Fig. 4, a significant increase in the FGF7 production amount was observed by adding L-ornithine at not less than 151 µM as compared with the control (P<0.05 or P<0.01).

It was confirmed from the above-mentioned results of Experimental Example 2 that ornithine has an action on hair papilla cells to promote the production of FGF7 which is a growth factor involved in the control of the hair cycle.

Therefore, the possibility was suggested that ornithine may promote FGF7 production by hair papilla cells, normalize the hair cycle by promoting transition from the telogen phase to the anagen phase of the hair cycle, and promote new hair growth.

### [Experimental Example 3] Study of influence of citrulline on proliferation of hair papilla cells

In the same manner as in Experimental Example 1, human hair papilla cells were cultured for 4 days, and seeded on a collagen-coated 96 well plate at a cell density of 2.5×10³ cells/0.1 mL/well. The cells were cultured for one day in a carbon dioxide incubator (5 volume% carbon dioxide, 37°C), and used for the evaluation of the proliferation of hair papilla cells. For the evaluation of the proliferation of hair papilla cells, the same evaluation medium as used in Experimental Example 1 was used.

L-citrulline was dissolved in the evaluation medium to prepare a solution.

In the above-mentioned hair papilla cell culture medium on the collagen-coated plate, the medium was replaced with the evaluation medium, the above-mentioned solution of L-citrulline was added to a concentration of 2 mM, and the cells were cultured for one day at 37°C, 5 volume% carbon dioxide (n=7).

Then the Cell Counting kit-8 (DOJINDO LABORATORIES) was added to each well by 10 µL, the plate was stood in a carbon dioxide incubator (5 volume% carbon dioxide, 37°C) for 2 hr, and the absorbance at 450 nm was measured using a microplate reader.

The case in which similar culture and treatment were performed without adding the solution of L-citrulline was used as the control.

The measurement results were converted to relative values (%) with the absorbance in the control as 100, and shown as mean±standard error in Fig. 5.

As shown in Fig. 5, a significant increase in the number of hair papilla cells was observed by adding L-citrulline at 2 mM as compared with the control (P<0.01).

It was confirmed from the above-mentioned results of Experimental Example 3 that citrulline promotes the proliferation of hair papilla cells.

Therefore, the possibility was suggested that citrulline may promote the proliferation of hair papilla cells which regulate hair growth, activate hair matrix cells, promote hair thickening, and grow long and thick hair, by increasing VEGF secretion.

### [Experimental Example 4] Study of influence of ornithine on proliferation of hair papilla cells

In the same manner as in Experimental Example 1, human hair papilla cells were cultured for 4 days, and seeded on a collagen-coated 96 well plate at a cell density of 2.5×10³ cells/0.1 mL/well. The cells were cultured for one day in a carbon dioxide incubator (5 volume% carbon dioxide, 37°C), and used for the evaluation of the proliferation of hair papilla cells. For the evaluation of the proliferation of hair papilla cells, a medium having the same amino acid composition as the evaluation medium used in Experimental Example 1 and other components diluted at a 1/4 concentration was used as the evaluation medium.

L-ornithine was dissolved in the above-mentioned evaluation medium to prepare a solution.

In the above-mentioned hair papilla cell culture medium on the collagen-coated plate, the medium was replaced with the evaluation medium, the above-mentioned solution of L-ornithine was added to a given concentration, and the cells were cultured for one day at 37°C, 5 volume% carbon dioxide (n=6 at each concentration).

Then the Cell Counting kit-8 (DOJINDO LABORATORIES) was added to each well by 10 µL, the plate was stood in a carbon dioxide incubator (5 volume% carbon dioxide, 37°C) for 2 hr, and the absorbance at 450 nm was measured using a microplate reader.

The case in which similar culture and treatment were performed without adding the solution of L-ornithine was used as the control.

The measurement results were converted to relative values (%) with the absorbance in the control as 100, and shown as mean±standard error in Fig. 6.

As shown in Fig. 6, a significant increase in the number of hair papilla cells was observed by adding L-ornithine at 756 µM or 3.7 mM as compared with the control (P<0.05 or P<0.01).

It was confirmed from the above-mentioned results of Experimental Example 4 that ornithine promotes the proliferation of hair papilla cells.

Therefore, the possibility was suggested that ornithine may promote the proliferation of hair papilla cells which regulate hair growth, promote hair thickening, and grow long and thick hair, in addition to increase of FGF7 secretion.

### [Experimental Example 5] Use test of citrulline and ornithine in human

As in the following, use test of citrulline and ornithine in human was conducted as described below.

### (1) Test subject

The subjects of the test were healthy males aged 20 to 59 and females aged 40 to 59 who had concerns about hair. Those who were taking citrulline, those who were taking ornithine, those who were outpatient to the hospital or taking medication for the treatment of diseases including hair-related diseases, those who were pregnant or breastfeeding, and those who had a plan to be pregnant or give birth were excluded from the test subjects.

### (2) Test method

"Kyowa Hakko Bio Citrulline" (220 mg per granule, citrulline content=800 mg/6 granules) and "Kyowa Hakko Bio Ornithine" (250 mg per granule, ornithine content=800 mg/6 granules) (Kyowa Hakko Bio Co., Ltd.) as the test foods were orally ingested by the test subjects for 12 weeks (6 granules each, once a day, every day between after dinner and before going to bed).

The test design was a single-group open test, and the test subjects compared and evaluated the conditions before and after ingestion of the test food. The aforementioned evaluation was conducted by answering the questionnaire shown in Table 2.

As shown in Table 2, the questionnaire had hair-related evaluation items (Q1 to Q12) as the main evaluation items, and 11 point scores of 0 to 10 were set for all evaluation items. The score before ingestion of the test food was set to 5, and after ingestion of the test food, the test subjects subjectively selected and reported the score at a frequency of once every two weeks.

**[Table 2]**

| | question No. and item | | score setting (condition before test food ingestion is 5) | | |
|---|---|---|---|---|---|
| | | | 0 1 2 3 4 5 6 7 8 9 10 | | |
| primary evaluation item | Q1 | whole hair | reduced | ---------- | increased |
| | Q2 | degree of worry of thinning hair | worried | ---------- | not worried |
| | Q3 | amount of newly grown hair | reduced | ---------- | increased |
| | Q4 | amount of fallen hair | increased | ---------- | reduced |
| | Q5 | amount of hair adhered to the pillow when waking up | increased | ---------- | reduced |
| | Q6 | amount of hair fallen out during hair washing | increased | ---------- | reduced |
| | Q7 | hair length growth | decelerated | ---------- | accelerated |
| | Q8 | amount of gray hair | increased | ---------- | reduced |
| | Q9 | thickness of hair | thinned | ---------- | thickened |
| | Q10 | volume of hair | reduced | ---------- | increased |
| | Q11 | itchiness of scalp | itching | ---------- | no itching |
| | Q12 | conspicuous head skin | conspicuous | ---------- | not conspicuous |
| secondary evaluation item | Q13 | skin dryness | dry | ---------- | not dry |
| | Q14 | skin shine | shiny | ---------- | not shiny |
| | Q15 | feeling of fatigue when waking up | remain | ---------- | not remain |
| | Q16 | sleep condition | unable to sleep | ---------- | can sleep |
| | Q17 | cold hands and feet | cold | ---------- | not cold |

### (3) Analysis of evaluation results

The population (PPS) excluding those whose ingestion rate of the test food was 50% or less and those who did not respond to the questionnaire was the subject of data analysis. Analysis was performed using the analysis soft "Microsoft Excel 2016, GraphPad Prism 6".

Regarding the response results of the questionnaire, the score before test food ingestion was subtracted from the score after 2, 4, 6, 8, 10, and 12 weeks after the test food ingestion, the amount of change over time was calculated, and comparison with that before test food ingestion was performed using the Kruskal-Wallis test. In addition, with regard to the amount of change obtained by subtracting the score before test food ingestion from the score 12 weeks after the test food ingestion, comparison between before and after test food ingestion was performed using the Wilcoxon signed rank test. The significance level was less than 5% in a two-tailed test, and a trend was set at between not less than 5% and less than 10%.

### (4) Evaluation results

Of the test subjects whose ingestion rate of the test food exceeded 50%, the above-mentioned analysis was performed on 31 subjects who responded to the questionnaire. No subject discontinued the test due to adverse events or exacerbation of health conditions.

For each evaluation item relating to hair, the amount of change in score over time (the amount of change from before test food ingestion, the amount of change in score before test food ingestion is set to 0) is shown in Table 3.

In addition, the amount of change in score at the end of test food ingestion was calculated and shown in Fig. 7 as mean±standard deviation. If no response was obtained after 12 weeks, which corresponds to after the test food ingestion, the amount of change in score was calculated using the response results at after 10 weeks.

**[Table 3]**

| | | whole (n=31) | number of response | p-value |
|---|---|---|---|---|
| Q1 (whole hair) | 2 weeks | 0.0 ± 0.2 | 30 | NS |
| | 4 weeks | 0.2 ± 0.8 | 31 | NS |
| | 6 weeks | 0.3 ± 0.6 | 29 | NS |
| | 8 weeks | 0.4 ± 0.6 | 28 | p<0.05 |
| | 10 weeks | 0.5 ± 0.8 | 29 | p<0.01 |
| | 12 weeks | 0.5 ± 0.7 | 25 | p<0.05 |
| Q2 (degree of worry of thinning hair) | 2 weeks | 0.0 ± 0.3 | 31 | NS |
| | 4 weeks | 0.2 ± 0.8 | 31 | NS |
| | 6 weeks | 0.3 ± 0.5 | 31 | NS |
| | 8 weeks | 0.3 ± 0.5 | 28 | p<0.1 |
| | 10 weeks | 0.4 ± 0.8 | 28 | p<0.01 |
| | 12 weeks | 0.3 ± 0.7 | 24 | NS |
| Q3 (amount of newly grown hair) | 2 weeks | 0.0 ± 0.2 | 30 | NS |
| | 4 weeks | 0.2 ± 0.4 | 31 | NS |
| | 6 weeks | 0.2 ± 0.5 | 30 | NS |
| | 8 weeks | 0.2 ± 0.4 | 28 | NS |
| | 10 weeks | 0.3 ± 0.6 | 29 | p<0.01 |
| | 12 weeks | 0.3 ± 0.6 | 24 | p<0.1 |
| Q4 (amount of fallen hair) | 2 weeks | 0.0 ± 0.5 | 31 | NS |
| | 4 weeks | 0.1 ± 0.8 | 31 | NS |
| | 6 weeks | 0.4 ± 0.7 | 30 | p<0.05 |
| | 8 weeks | 0.4 ± 0.8 | 28 | NS |
| | 10 weeks | 0.6 ± 0.9 | 29 | p<0.05 |
| | 12 weeks | 0.5 ± 0.8 | 23 | p<0.05 |
| Q5 (amount of hair adhered to pillow when waking up) | 2 weeks | 0.1 ± 0.3 | 31 | NS |
| | 4 weeks | 0.2 ± 0.6 | 31 | NS |
| | 6 weeks | 0.3 ± 0.5 | 31 | p<0.1 |
| | 8 weeks | 0.4 ± 0.7 | 28 | p<0.1 |
| | 10 weeks | 0.6 ± 1.1 | 29 | p<0.05 |
| | 12 weeks | 0.7 ± 0.9 | 24 | p<0.001 |
| Q6 (amount of hair fallen out during hair washing) | 2 weeks | 0.0 ± 0.5 | 31 | NS |
| | 4 weeks | 0.3 ± 0.9 | 31 | p<0.1 |
| | 6 weeks | 0.3 ± 0.8 | 31 | NS |
| | 8 weeks | 0.3 ± 0.8 | 28 | NS |
| | 10 weeks | 0.7 ± 1.0 | 29 | p<0.01 |
| | 12 weeks | 0.5 ± 0.9 | 22 | p<0.05 |
| Q7 (hair length growth) | 2 weeks | 0.0 ± 0.2 | 31 | NS |
| | 4 weeks | 0.1 ± 0.3 | 31 | NS |
| | 6 weeks | 0.2 ± 0.4 | 30 | NS |
| | 8 weeks | 0.1 ± 0.4 | 27 | NS |
| | 10 weeks | 0.3 ± 0.6 | 29 | p<0.1 |
| | 12 weeks | 0.3 ± 0.9 | 24 | NS |
| Q8 (amount of gray hair) | 2 weeks | 0.0 ± 0.5 | 31 | NS |
| | 4 weeks | 0.2 ± 0.7 | 31 | NS |
| | 6 weeks | 0.1 ± 0.6 | 31 | NS |
| | 8 weeks | 0.0 ± 0.4 | 28 | NS |
| | 10 weeks | 0.1 ± 0.6 | 29 | NS |
| | 12 weeks | 0.0 ± 0.6 | 24 | NS |
| Q9 (thickness of hair) | 2 weeks | 0.1 ± 0.2 | 31 | NS |
| | 4 weeks | 0.2 ± 0.6 | 31 | NS |
| | 6 weeks | 0.2 ± 0.5 | 31 | NS |
| | 8 weeks | 0.1 ± 0.4 | 28 | NS |
| | 10 weeks | 0.4 ± 0.9 | 29 | p<0.05 |
| | 12 weeks | 0.3 ± 0.6 | 23 | p<0.05 |
| Q10 (volume of hair) | 2 weeks | 0.2 ± 0.5 | 31 | NS |
| | 4 weeks | 0.3 ± 0.8 | 31 | NS |
| | 6 weeks | 0.2 ± 0.4 | 31 | NS |
| | 8 weeks | 0.3 ± 0.5 | 27 | NS |
| | 10 weeks | 0.6 ± 0.9 | 29 | p<0.01 |
| | 12 weeks | 0.5 ± 0.8 | 24 | p<0.01 |
| Q11 (itchiness of scalp) | 2 weeks | 0.1 ± 0.5 | 31 | NS |
| | 4 weeks | 0.2 ± 0.6 | 31 | NS |
| | 6 weeks | 0.1 ± 0.8 | 31 | NS |
| | 8 weeks | 0.2 ± 0.7 | 28 | NS |
| | 10 weeks | 0.3 ± 0.7 | 29 | NS |
| | 12 weeks | 0.5 ± 1.1 | 24 | NS |
| Q12 (conspicuous head skin) | 2 weeks | 0.0 ± 0.2 | 31 | NS |
| | 4 weeks | 0.0 ± 0.7 | 30 | NS |
| | 6 weeks | 0.1 ± 0.5 | 31 | NS |
| | 8 weeks | 0.1 ± 0.3 | 27 | NS |
| | 10 weeks | 0.3 ± 0.7 | 29 | p<0.05 |
| | 12 weeks | 0.3 ± 0.7 | 24 | NS |

As shown in Table 3, it was observed that the amount of change in score for Q1 (amount of hair) increased significantly on and after 8 weeks from the test food ingestion, and the number of test subjects who felt that the amount of hair increased due to the test food ingestion increased.

It was observed that the amount of change in the score for Q2 (degree of worry of thinning hair) tended to increase after 8 weeks, a significant increase was observed after 10 weeks, and the number of test subjects who did not worry about thinning hair increased.

It was observed that the amount of change in score for Q3 (amount of newly grown hair) showed a significant increase after 10 weeks, tended to increase after 12 weeks, and the number of test subjects increased who felt that the amount of newly grown hair increased.

It was observed that the amount of change in score for Q4 (amount of fallen hair) increased significantly on and after 6 weeks and 10 weeks, and the number of test subjects increased who felt that the amount of fallen hair decreased.

It was observed that the scores for Q5 (amount of hair adhered to the pillow when waking up), Q6 (amount of hair fallen out during hair washing), Q9 (thickness of hair), and Q10 (volume of hair) each significantly increased on and after 10 weeks, and the test subjects who felt that fallen hair decreased and the test subjects who felt that the hair thickened increased.

It was shown that the amount of change in score for Q12 (conspicuous head skin) increased significantly 10 weeks after the test food ingestion, and the number of test subjects increased who felt that the skin became less conspicuous.

The amount of change in score for Q7 (hair length growth) tended to increase only after 10 weeks.

No significant change was observed in the amount of change in scores for Q8 (amount of gray hair) and Q11 (itchiness of scalp). However, positive values were obtained, and the presence of test subjects was observed who felt that gray hair and itchiness of the scalp decreased.

As shown in Fig. 7, the amount of change in the score significantly increased in the evaluation items other than Q8 (significant at P<0.001 for Q1, Q4 to Q6, and Q10, significant at P<0.01 for Q2, Q3, and Q9, significant at P<0.05 for Q7, Q11, and Q12) as compared with before the test food ingestion (pre), and the usefulness of test food ingestion for worry of hair was suggested.

From the above-mentioned results of Experimental Example 5, it was suggested that oral ingestion of citrulline and ornithine makes hair grow thicker and is effective in improving thinning hair and fallen hair.

### [Experimental Example 6] Study of influence of content ratio of citrulline and ornithine

In an animal experiment using male mice, the influence of the content ratio of citrulline and ornithine during oral administration on the plasma concentration of citrulline was studied as follows.

### (1) Experimental method

(i) 5-Week-old male C3H/HeN mice (CLEA Japan, Inc.) were received and raised in an animal breeding room under Specific Pathogen Free (SPF). After providing an acclimation period, the mice were divided into groups at the time of 7 weeks of age and subjected to the test. The mice were reared in polycages (small) with paper bedding (pepper clean), one per cage, and freely given solid feed CRF1 (Oriental Yeast Co., Ltd.) and tap water. The breeding room was set to 12 hours light and 12 hours dark (lighting: 7:00 to 19:00), temperature 23±1°C, and humidity 55±10%.

### (ii) Preparation of test solution

As the test solutions, L-citrulline aqueous solution (0.2 g/mL) and L-ornithine hydrochloride aqueous solution (0.2 g/mL) were prepared using L-citrulline (Nacalai Tesque, Inc.: Cat No. 09113-31), L-ornithine hydrochloride (Nacalai Tesque, Inc., Ltd.: Cat No. 25718-92), and water for injection (Otsuka Pharmaceutical Factory, Inc.), and a mixed solution of citrulline and ornithine hydrochloride was prepared as shown in Table 4, and they were used as test solutions.

**[Table 4]**

| L-citrulline:L-ornithine hydrochloride weight ratio (molar ratio) | citrulline solution amount (mL) | ornithine solution amount (mL) | distilled water amount (mL) |
|---|---|---|---|
| 1:0(1:0) | 0.5 | 0 | 0.5 |
| 1:0.15(1:0.16) | 0.5 | 0.075 | 0.425 |
| 1:0.3(1:0.31) | 0.5 | 0.15 | 0.35 |
| 1:0.6(1:0.62) | 0.5 | 0.3 | 0.2 |
| 1:1(1:1.04) | 0.5 | 0.5 | 0 |

### (iii) Administration of test solution and measurement of plasma concentration

The mice were divided into 6 groups (10 to 15 mice/group) and, with the day on which the grouping was performed as day 1, each test solution was orally administered to each group at 10 mL/kg once a day for 5 days from day 1 to day 5. As a control, the same amount of distilled water was administered. The heart blood was collected under isoflurane anesthesia at 30 min, 1 hr, and 2 hr after administration on day 5, and the amino acid concentration of the separated plasma was measured using UF-Amino Station (Shimadzu Corporation).

### (iv) Statistical analysis of measurement results

The statistical analysis of the measurement results was performed using GraphPad Prism 6 (GraphPad Software, Inc.). Significant digits after the decimal point were 2 digits, and a significant difference test of blood concentration after elapse of each time was performed at p<0.05 using the unpaired Tukey's multiple comparison test, for each group except the distilled water administration group (DW).

### (2) Results

Fig. 8 shows the measurement results of the plasma concentration of citrulline at 30 min, 1 hr, and 2 hr after administration as mean±standard deviation.

As shown in Fig. 8, it was observed that the citrulline plasma concentration 30 min after administration was significantly high in a test solution (molar ratio=1:0.16) administration group in which the content ratio of citrulline and ornithine hydrochloride (citrulline:ornithine hydrochloride) was 1:0.15 (weight ratio), and a test solution (molar ratio=1:0.31) administration group in which the content ratio was 1:0.3 (weight ratio), as compared with a test solution (molar ratio=1:0.62) administration group in which the content ratio was 1:0.6 (weight ratio), and a test solution (molar ratio=1:1.04) administration group in which the content ratio was 1:1 (weight ratio) (A).

It was observed that the citrulline plasma concentration 1 hr after administration was significantly high in a test solution (molar ratio=1:0) administration group in which the content ratio of citrulline and ornithine hydrochloride (citrulline:ornithine hydrochloride) was 1:0 (weight ratio), a test solution (molar ratio=1:0.16) administration group in which the content ratio was 1:0.15 (weight ratio), and a test solution (molar ratio=1:0.31) administration group in which the content ratio was 1:0.3 (weight ratio), as compared with a test solution (molar ratio=1:0.62) administration group in which the content ratio was 1:0.6 (weight ratio), and a test solution (molar ratio=1:1.04) administration group in which the content ratio was 1:1 (weight ratio) (B).

It was observed that the citrulline plasma concentration 2 hr after administration was significantly high in a test solution (molar ratio=1:0) administration group in which the content ratio of citrulline and ornithine hydrochloride (citrulline:ornithine hydrochloride) was 1:0 (weight ratio), and a test solution (molar ratio=1:0.16) administration group in which the content ratio was 1:0.15 (weight ratio), as compared with a test solution (molar ratio=1:1.04) administration group in which the content ratio was 1:1 (weight ratio), and also significantly high in a test solution (molar ratio=1:0.16) administration group in which the content ratio was 1:0.15 (weight ratio) as compared with a test solution (molar ratio=1:0.62) administration group in which the content ratio was 1:0.6 (weight ratio) (C).

In addition, Fig. 9 shows the measurement results of the plasma concentration of ornithine at 30 min, 1 hr, and 2 hr after administration as mean±standard deviation.

As shown in Fig. 9, it was observed that the plasma concentration of ornithine at 30 min and 1 hr after administration was significantly high in an ornithine content ratio dependent manner (A, B).

It was observed that the ornithine plasma concentration 2 hr after administration was significantly high in a test solution (molar ratio=1:1.04) administration group in which the content ratio of citrulline and ornithine hydrochloride (citrulline:ornithine hydrochloride) was 1:1 (weight ratio) as compared with a test solution (molar ratio=1:0) administration group in which the content ratio was 1:0 (weight ratio), and a test solution (molar ratio=1:0.31) administration group in which the content ratio was 1:0.3 (weight ratio).

From the above-mentioned results of Experimental Example 6, it was suggested that citrulline absorption is effectively improved, and hair thickening promoting effect and hair cycle normalization promoting effect, and further, thinning hair or hair loss preventing or improving effect are further improved, when the content ratio of citrulline and ornithine (citrulline:ornithine) is 1:0.16 - 1:0.31 in molar ratio.

### [Industrial Applicability]

As described in detail above, according to the present invention, a composition for promoting hair thickening can be provided.

The composition for promoting hair thickening of the present invention can promote hair thickening by promoting VEGF or FGF7 production in hair papilla cells and proliferation of hair papilla cells, and grow long, thick, and healthy hair.

According to the present invention, moreover, a composition for maintaining the anagen phase of the hair cycle and a composition for promoting VEGF production can be provided.

The composition for maintaining the anagen phase of the hair cycle of the present invention can promote division and proliferation of hair matrix cells, suppress shortening of the anagen phase of hair, and normalize the hair cycle by maintaining the normal anagen phase, by promoting VEGF production in hair papilla cells.

The composition for promoting VEGF production of the present invention can promote VEGF production in hair papilla cells, promote division and proliferation of hair matrix cells, suppress shortening of the anagen phase of hair, normalize the hair cycle by maintaining the normal anagen phase, and promote thickening of hair by promoting proliferation of hair papilla cells.

Furthermore, according to the present invention, a composition for promoting transition from the telogen phase to the anagen phase of the hair cycle and a composition for promoting FGF7 production can be provided.

The composition for promoting transition from the telogen phase to the anagen phase of the hair cycle of the present invention can shorten the telogen phase which is from the degeneration of the hair root until a hair begins to grow anew, normalize the hair cycle by promoting the transition from the telogen phase to the anagen phase, promote the new hair growth by promoting division and proliferation of hair matrix cells, by promoting FGF7 production in hair papilla cells.

The composition for promoting FGF7 production of the present invention can shorten the telogen phase which is from the degeneration of the hair root until a hair begins to grow anew by promoting FGF7 production in dermal papilla cells, normalize the hair cycle by promoting the transition from the telogen phase to the anagen phase, and promote the new hair growth by promoting the division and proliferation of hair matrix cells.

The composition for promoting hair thickening, the composition for maintaining the anagen phase of the hair cycle, and the composition for promoting VEGF production of the present invention can be preferably used as hair-growing agents. The composition for promoting transition from the telogen phase to the anagen phase of the hair cycle and the composition for promoting FGF7 production of the present invention can be preferably used as new-hair-growing agents.

Furthermore, according to the present invention, a composition for promoting normalization of the hair cycle can be provided.

The composition for promoting the normalization of the hair cycle of the present invention can normalize the hair cycle by either or both of the action of maintaining the anagen phase of the hair cycle by promoting VEGF production, and the action of promoting transition from the telogen phase to the anagen phase of the hair cycle by promoting FGF7 production.

The composition for promoting hair thickening, the composition for maintaining the anagen phase of the hair cycle, and the composition for promoting VEGF production of the present invention, the composition for promoting transition from the telogen phase to the anagen phase of the hair cycle and the composition for promoting FGF7 production of the present invention, and the composition for promoting normalization of the hair cycle of the present invention can favorably improve or prevent thinning hair or hair loss, and are particularly useful for androgenetic alopecia and female pattern alopecia.

This application is based on a patent application No. 2020-056850 filed in Japan, the contents of which are incorporated in full herein.

## Claims

1. A composition for promoting hair thickening, comprising one or more members selected from the group consisting of citrulline, tryptophan, glutamine, and ornithine.

2. The composition according to claim 1, wherein a total content of one or more members selected from the group consisting of citrulline, tryptophan, glutamine, and ornithine is 0.1 µM to 1 M.

3. The composition according to claim 1 or 2, wherein the composition comprises one or more members selected from the group consisting of citrulline, tryptophan, and glutamine, and ornithine.

4. The composition according to claim 3, wherein a content ratio of one or more members selected from the group consisting of citrulline, tryptophan, and glutamine, and ornithine (one or more members selected from the group consisting of citrulline, tryptophan, and glutamine:ornithine) is 1:0.1 to 1:0.5 in molar ratio.

5. A composition for maintaining the anagen phase of the hair cycle, comprising one or more members selected from the group consisting of citrulline, tryptophan, and glutamine.

6. The composition according to claim 5, wherein a total content of one or more members selected from the group consisting of citrulline, tryptophan, and glutamine is 0.1 µM to 1 M.

7. A composition for promoting VEGF production, comprising one or more members selected from the group consisting of citrulline, tryptophan, and glutamine.

8. The composition according to claim 7, wherein a total content of one or more members selected from the group consisting of citrulline, tryptophan, and glutamine is 0.1 µM to 1 M.

9. A composition for promoting transition from the telogen phase to the anagen phase of the hair cycle, comprising ornithine.

10. The composition according to claim 9, wherein a content of ornithine is 1 µM to 1 M.

11. A composition for promoting FGF7 production, comprising ornithine.

12. The composition according to claim 11, wherein a content of ornithine is 1 µM to 1 M.

13. A composition for promoting normalization of the hair cycle, comprising one or more members selected from the group consisting of citrulline, tryptophan, glutamine, and ornithine.

14. The composition according to claim 13, wherein a total content of one or more members selected from the group consisting of citrulline, tryptophan, glutamine, and ornithine is 0.1 µM to 1 M.

15. The composition according to claim 13 or 14, wherein the composition comprises one or more members selected from the group consisting of citrulline, tryptophan, and glutamine, and ornithine.

16. The composition according to claim 15, wherein a content ratio of one or more members selected from the group consisting of citrulline, tryptophan, and glutamine, and ornithine (one or more members selected from the group consisting of citrulline, tryptophan, and glutamine:ornithine) is 1:0.1 to 1:0.5 in molar ratio.

17. A food for preventing or improving thinning hair or hair loss, comprising either or both of the composition for promoting VEGF production according to claim 7 or 8 and the composition for promoting FGF7 production according to claim 11 or 12.

18. An external composition for preventing or improving thinning hair or hair loss, comprising either or both of the composition for promoting VEGF production according to claim 7 or 8 and the composition for promoting FGF7 production according to claim 11 or 12.

19. The external composition according to claim 18, wherein the composition is a skin external quasi-drug.

20. The external composition according to claim 18, wherein the composition is a hair cosmetic or a scalp cosmetic.

21. The composition according to any one of claims 1 to 16, wherein the composition is a composition for suppressing or ameliorating scalp itchiness.
